# EUROPEAN PATENT SPECIFICATION

(11) **EP 2 515 918 B1**
(45) Date of publication and mention of the grant of the patent: **08.10.2014**
(21) Application number: 10842480.5
(22) Date of filing: 13.12.2010
(51) Int. Cl.: A61K 33/38, A61K 9/20

(54) **SMOKING CESSATION LOZENGE CONTAINING TOBACCO ALKALOID AND SILVER SALT**
RAUCHERENTWÖHNUNGSLUTSCHTABLETTE MIT EINEM TABAKALKALOID UND EINEM SILBERSALZ
PASTILLE DE DÉSACCOUTUMANCE DU TABAC CONTENANT UN ALCALOÏDE DU TABAC ET UN SEL D'ARGENT

(30) Priority: 21.12.2009 US 288356 P
(43) Date of publication of application: 31.10.2012
(73) Proprietor: RCP Development, Inc., Sarasota, FL 34243 (US)
(72) Inventor: WRIGHT IV, Curtis, Gloucester, Massachusetts 01930 (US)
(74) Representative: Wilson, Justin Scott
(86) International application number: PCT/US2010/060042
(87) International publication number: WO 2011/084376

(56) References cited:
- US-A- 4 832 994
- US-A- 5 032 612
- US-A- 5 662 920
- US-A- 6 082 368
- US-A1- 2004 101 543
- US-A1- 2009 196 834
- US-B2- 6 668 839
- US-B2- 6 845 777

## Description

### DESCRIPTION OF RELATED ART

There is a developing market for smoking cessation aids. Some approaches have been to deliver nicotine via transdermal or transmucosal devices, which allow delivery of nicotine through the skin or mouth, respectively. U.S. Patent 5,512,306 describes a smoking cessation aid in the form of an inclusion complex formed between nicotine and a cyclo compound such as polysaccharide. U.S. Patent 5,525,351 discloses a saliva-soluble stimulant formed from a gel and nicotine.

Another approach has been to discourage individuals from smoking by administering compounds, such as silver salts, that eliminate or diminish the pleasurable aspects of smoking. When an individual smokes a cigarette after consuming the silver salt, for example, the cigarette smoke has an unpleasant taste. U.S. Patents 4,832,994 and 4,980,172 describe formulations containing silver acetate in a lozenge, gum, spray or mouthwash.

Existing smoking cessation treatments generally suffer from a high rate of relapse, particularly in the early stages of treatment. There remains a need for more effective smoking cessation therapies.

### SUMMARY

In one embodiment, a smoking cessation aid comprises a lozenge containing powdered tobacco and a therapeutically effective amount of a silver salt.

In a second embodiment, a smoking cessation aid comprises a lozenge containing nicotine and a therapeutically effective amount of a silver salt.

In a third embodiment, a smoking cessation aid comprises a lozenge containing (i) at least one of anatabine, anabasine, and nornicotine, and (ii) a therapeutically effective amount of a silver salt.

By combining appropriate amounts of a silver salt and tobacco powder or tobacco alkaloids in a suitable lozenge, a more effective smoking cessation aid may be prepared. The improved efficacy of the smoking cessation aid results from the lozenge having a unique combination of a pleasant taste, delivering nicotine and/or other tobacco alkaloids, and delivering an active agent for discouraging smoking.

### BRIEF DESCRIPTION OF THE DRAWINGS

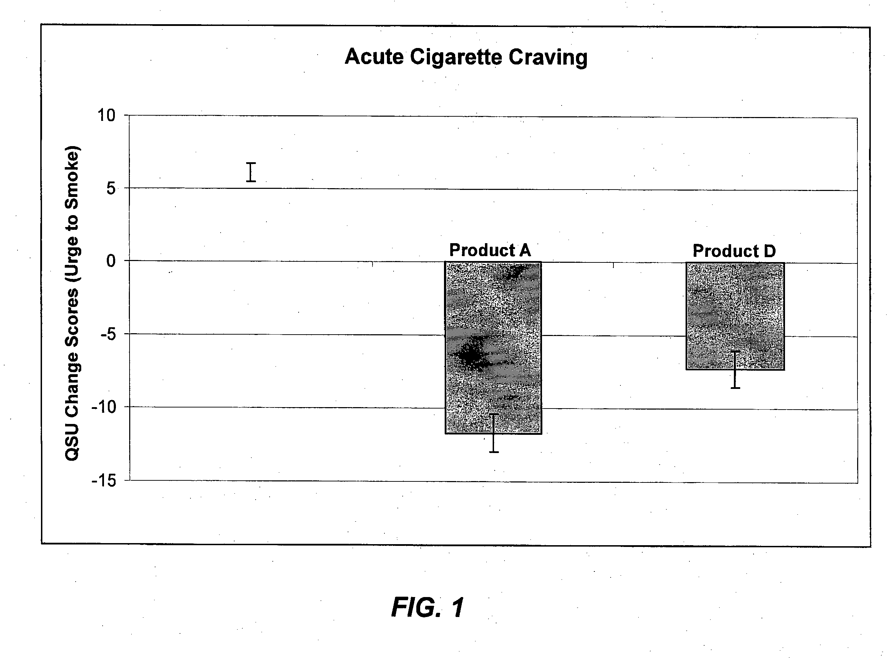

FIG. 1 is a graph showing subjects' decrease in craving for smoking following administration of a lozenge containing powdered tobacco and silver acetate ("Product A") and a lozenge containing only silver acetate ("Product D").

### DETAILED DESCRIPTION OF THE INVENTION

In a first embodiment, a smoking cessation aid comprises a lozenge containing powdered tobacco and a therapeutically effective amount of a silver salt. In a second embodiment, a smoking cessation aid comprises a lozenge containing nicotine and a therapeutically effective amount of a silver salt. In a third embodiment, a smoking cessation aid comprises a lozenge containing (i) at least one of anatabine, anabasine, and nornicotine, and (ii) a therapeutically effective amount of a silver salt.

The silver salt may be any one or more silver compounds that are capable of delivering silver ions in the oral cavity, such as silver acetate or silver lactate. Silver acetate is advantageous due to its water-solubility. The smoking cessation aid usually contains from about 1 to about 10 mg, often from about 2 to about 8 mg, of silver acetate. For example, the lozenge may contain about 2 mg, about 3 mg, about 4 mg, about 5 mg, about 6 mg, about 7 mg, about 8 mg, or about 9 mg of a silver salt such as silver acetate. It was found that these relatively low silver acetate concentrations provide an acceptable taste for the smoking cessation aid, yet at the same time are effective for discouraging smoking by imparting an unfavorable taste to the user when a tobacco product is smoked, e.g., for about 60 minutes after consuming the smoking cessation aid.

In a first embodiment, the smoking cessation aid contains powdered tobacco and optionally other ingredients such as binders. The amount of powdered tobacco may range, for example, from about 5 to about 500 mg, and often ranges from about 35 to about 250 mg. Other components, such as flavorants, may also be present in suitable quantities. The smoking cessation aid usually contains primarily water-soluble (or saliva-soluble) components, permitting transdermal or transmucosal delivery of nicotine, silver acetate, and other components present. The powder is typically milled fine enough so that even insoluble components can be easily swallowed.

The powdered tobacco may be produced from cured tobacco stems, lamina, or both (hereinafter collectively referred to as "tobacco material"). The relative proportion of tobacco material in the product depends on such factors as the particular composition of the tobacco leaf. The lozenge most often has from about 10 % to about 80 % of powdered tobacco by weight, more usually from about 25 % to about 55 % by weight.

Preferably, the cured tobacco material is pulverized, e.g. milled, to form a powdered tobacco. In this manner, the tobacco material is milled fine enough to produce an easily swallowed product. Alternatively, an extract of the tobacco material is dried to form a powder. In the extraction process, cured tobacco material is extracted with a solvent, typically water, ethanol, steam, or carbon dioxide. The resulting solution contains the soluble components of the tobacco, including nicotine. The solution is then dried and ground, as needed, to form a powder.

Powdered tobacco may then be used to form a lozenge. Prior to forming the lozenge it may be desirable to process the powdered tobacco to form larger particles, such as by granulation or by rolling and grinding. Such processes provide particles, which are more readily formed into lozenges, and form lozenges, which do not disintegrate during handling and in the package. The larger particles are easier to handle than the smaller particles and do not produce the "dust" associated with small powder particles. Furthermore, the larger particles compress into lozenges more readily than powder particles. This allows for higher speed lozenge formulation and easier machining of the lozenges. In addition, using either granulation or rolling and pressing provides an even distribution of flavorants, coloring agents, and the like, throughout the final lozenge.

Granulation increases the particle size by adding a binder to the powder and allowing the powder to clump into larger particles. By using a fluid granulation process, for example, the powder clumps into fairly larger particles. The granulation process may also be used to add the flavorants or other ingredients to the particles by including dissolved flavorants in the binder solution.

Rolling under pressure presses the particles into a flake or a bark. The flake or bark is then ground to form particles, which are larger than the original powder particles. Prior to rolling, the powder may be mixed with other ingredients including binders and flavorants.

The powder or particles are then compressed to form a lozenge. The lozenge may be processed and packaged by any suitable means. During use, the lozenge typically is placed in the mouth and allowed to dissolve, releasing the nicotine, silver acetate, and other tobacco components. Any material that does not dissolve is easily swallowed along with the dissolved components. That is, for example, a lozenge formed from whole leaf pulverized tobacco, will disintegrate and dissolve in the mouth, such that any insoluble components are in the form of very small particles that are easily swallowed with the saliva.

Flue (bright) varieties of tobacco are often used, i. e., Virginia flue. Other tobacco varieties may be used, such as Burley, dark-fired, or other commercial tobacco varieties. If desired, two or more tobacco varieties may be combined to form a blend. The powdered tobacco may be formed from cured tobacco stems, lamina, or both. Tobacco stems generally have higher amounts of fibrous components than are present in lamina. Other differences exist. For example, stems typically have less bitterness than lamina. Lamina generally is easier to mill and has higher concentrations of soluble components.

First, tobacco is grown and harvested. The tobacco is cured and then removed from the curing barn. If only the stem or lamina is being used, the stem or lamina may be separated from the rest of the leaf either before or after curing. Preferably, the stem or lamina is separated after curing.

After curing, before or after milling or extracting, the tobacco material is preferably subjected to a sterilization technique. The sterilization technique typically irradiates the tobacco to destroy any microbes remaining on the tobacco. Any suitable radiation may be used such as, but not limited to, microwaves, gamma rays or electron beams. U.S. Patent 6,311,695 describes the use of electron beams.

The cured tobacco material is subjected to a process to form a powdered tobacco. The process may comprise extracting and drying, or a pulverizing process such as milling.

A preferred method of forming powdered tobacco is pulverizing the cured tobacco material into a powder. The cured tobacco material may be pulverized by any suitable process, preferably by milling. Preferably, the tobacco material is milled into particles having a particle size of about 50 to about 300 mesh, typically about 150 mesh.

The tobacco material may be chopped or powdered and then subjected to an extraction process with water or other aqueous solvent. With the exception of the pulp, substantially all of the components in tobacco are water-soluble, including alkaloids such as nicotine, nornicotine, anabasine, and anatabine.

Methods for forming aqueous tobacco extracts are known in the art as described, for example, in U.S. Patent 5,065,775. In general, tobacco material is contacted with an aqueous solution to extract soluble components. The time of contact will depend on such factors as the water to tobacco ratio and the temperature of the aqueous solution. The aqueous extract produced by contact with the water solution is then separated from the insoluble fibrous tobacco residue, which can be accomplished using conventional solid-liquid separation techniques. For example, squeezing, centrifugation, and filtration techniques may be employed. If necessary, the separated tobacco extract may then be treated to adjust soluble solids content.

More particularly, cured tobacco material is contacted with an aqueous extraction solvent. Contact can be performed in either a continuous or batch-wise manner. The mixture of tobacco material and extraction solvent can be agitated in order to enhance removal of water-soluble components from the tobacco material. The mixture is subjected to separation conditions (e.g., using a centrifuge) so as to provide an aqueous tobacco extract (*i.e*., a water-soluble tobacco extract within the extraction solvent), and a water-insoluble tobacco residue.

The aqueous extraction solvent is primarily water, normally at least about 90 wt% water, and can be essentially pure water such as deionized water, distilled water, or tap water. The extraction solvent can be a co-solvent mixture, such as a mixture of water and one or more solvents that are miscible therewith. An example of such a co-solvent mixture is a solvent containing 95 parts water and 5 parts ethanol per 100 parts by weight. The extraction solvent also may include substances such as pH adjusters (*i.e*., acids or bases) or pH buffers dissolved therein. For example, an aqueous solvent can have ammonium hydroxide or gaseous ammonia incorporated therein so as to provide a solvent having a pH of about 8 or more.

The amount of the tobacco material which is contacted with the extraction solvent can vary over a wide range and depends upon such factors as the type of solvent, the temperature at which the extraction is performed, the type or form of tobacco material which is extracted, the manner in which contact of the tobacco material and solvent is conducted, and the type of extraction process which is performed. Typically, for a batch-wise extraction, the weight of extraction solvent relative to the tobacco stems is greater than about 6:1, oftentimes greater than about 8:1 and in certain instances can be greater than about 12:1. The manner for contacting the tobacco material with the extraction solvent is not particularly critical, e.g., the tobacco material can be extracted in either a continuous or batch-wise manner. For example, the tobacco material can be extracted using a continuous counter-current extractor.

Tobacco material can be extracted in a batch-wise manner one or more times using the solvent. Normally, the weight of extract and solvent relative to the weight of tobacco material for each batch extraction ranges from about 6:1 to about 40:1, more often from about 15:1 to 25:1. The number of times that the tobacco stems is contacted batch-wise with the processed tobacco extract and solvent ranges from about 1 to about 8 times, more usually from about 3 to 5 times.

The tobacco material can be extracted continuously with water, ethanol, carbon dioxide, or steam as solvents. Normally, the weight of aqueous solvent relative to the tobacco material with which it is contacted during a continuous extraction process is greater than about 40:1 and often is greater than about 50:1. The conditions under which the extraction is performed can vary. Typical temperatures range from about -20 to 100 °C, more often from about 10 to 60 °C. Alternatively, steam can be used to extract the soluble components, which can be recovered in a condenser. The solvent/tobacco material mixture can be agitated (e.g., stirred, shaken or otherwise mixed) in order to increase the rate at which extraction occurs.

Typically, for a batch-wise extraction, adequate extraction of components occurs in less than about 60 minutes, oftentimes in less than about 30 minutes. A wide variety of components can be extracted from the tobacco material. Water-soluble tobacco components that are extracted from tobacco material using a solvent having an aqueous character include alkaloids (e.g., nicotine), acids, salts, sugars, and the like. Extracted tobacco components include many of the aroma-producing and flavorful substances of the tobacco material.

The solvent and tobacco extract are separated from the insoluble tobacco residue. The manner of separation can vary; however, it is convenient to employ conventional separation techniques involving the use of filters, centrifuges, screw presses, converging belts, rotating disk presses, and the like. The insoluble residue can be treated to remove additional solvent and tobacco extract therefrom.

The solvent and tobacco components extracted thereby optionally can be filtered to remove suspended insoluble particles. In some cases it may be desirable to adjust the pH of the aqueous tobacco extract. For example, as described in U.S. Patent 5,065,775, pH of an aqueous tobacco extract can be raised to promote removal of basic compounds, lowered to promote removal of acidic compounds, or made neutral to promote removal of neutral compounds.

After extraction, the aqueous extract is dried into a powder by any suitable process. Preferably the extract is spray-dried to form a powder. Spray-drying techniques are disclosed, for example, in U.S. Patent 5,387,416, the disclosure of which is hereby incorporated by reference in its entirety. The powder is optionally bleached and then dried. The powder generally has a particle size of below 80 mesh and typically between 100 and 300 mesh.

If the average particle size of the powder is smaller than 80 mesh, as is typically the result with the extraction process, and may be the result of the milling process, then the powder is subjected to a process to increase its particle size, to conglomerate particles to make larger particles or both, to an average size greater than 80 mesh, preferably to an average particle size of between 14 and 80 mesh. Any suitable process may be used to increase particle size. Preferably the powder is granulated, or rolled and ground. Granulating or rolling and grinding the powder forms particles, which are easier to handle, machine, and compress into lozenges than the powder.

The powder may be granulated in any suitable manner. A preferred method uses a fluid bed granulator. The powder is placed in a fluid bed product bowl in the chamber of the fluid bed granulator. Air or other suitable gas is introduced into the chamber to blow the powder around the chamber. A liquid solution containing at least a binder is introduced into the chamber in the form of a very fine mist. The particles blow around in the mist. The particles become coated and start to clump together to make discrete uniform particles. A second mist of a buffer solution may then be introduced. After spraying, the particles are dried to the desired moisture level and lubricants may be added to the particles.

The powder may contain only tobacco or may include other ingredients such as sweeteners, flavorants, coloring agents, and fillers. The liquid solution may simply contain a binder or may contain other ingredients in addition to the binder such as flavorants, coloring agents, sweeteners, and fillers. The lubricant may be a powder or a liquid. The ingredients may be distributed throughout the tobacco powder, binder solution, and lubricant.

The rolling and grinding process passes the powder though a roller under high pressure. The powder forms flake (bark), which is then ground to form particles having a size larger than the original particle size, i.e. greater than 80 mesh. Tobacco lozenges resulting from granulated or rolled and ground processes do not disintegrate but instead hold their form.

If desired, one or more flavorants may be added to the tobacco powder, such as peppermint, menthol, and wintergreen or spearmint. Wintergreen oil, or methyl salicylate, can be prepared by heating methanol and salicylic acid in the presence of sulfuric acid, or by distillation from the leaves of *Gaultheria procumbens* or the bark of *Betula lenta.* If desired, one or more additional flavorants, such as propolis, eucalyptus, and/or cinnamon, may be provided to reduce the irritation that can be caused by nicotine in the mouth and to enhance the flavor of the powdered tobacco while removing bitterness. U.S. Patent 5,845,647 describes the use of propolis in tobacco-containing chewing gum and other tobacco products. The amount of each flavorant typically ranges from about 0.5 to about 10 wt%, based on the total weight of the powdered tobacco.

Other ingredients may be added to the powder prior to forming into a lozenge. Such ingredients include, but are not limited to sweeteners, fillers, coloring agents, buffers, and lubricants. Such ingredients may be added to the powdered tobacco or, if using a granulation process, to the binder solution. The relative amounts of such other components can vary over a wide range, depending on such factors as the particular tobacco used and consumer preferences. Typically, the amounts of individual components will range from about 0.5 wt% to about 10 wt%, based on the total weight of the powdered tobacco.

The smoking cessation aid can be prepared by any suitable technique and is not limited by any particular method for its production. For example, powdered tobacco can be combined with excipients and a binder, and then granulated. The granulation can be dry-blended with the remaining ingredients, and compressed into a lozenge. The percent by weight of tobacco in the lozenge will vary depending on such factors as whether tobacco lamina is used. Since lamina has a higher concentration of nicotine than stems, generally lower amounts of tobacco are employed when lamina is used and higher amounts of tobacco are employed when only stems are used. The lozenge usually contains from about 10 to 80 wt% of powdered tobacco, often about 25 to 55 wt%. The weight of the lozenge can vary over a wide range, most often from about 75 mg to about 1,000 mg, more usually from about 150 mg to about 550 mg.

The user may consume the lozenge by placing it in the mouth. As the lozenge dissolves, the active tobacco components are dissolved in the saliva. Components in the powdered tobacco will transmucously absorb into the mouth and/or will be easily swallowed with the saliva.

In a second embodiment, a smoking cessation aid comprises a lozenge containing nicotine and a therapeutically effective amount of a silver salt. As in the first embodiment, the amount of silver salt typically ranges from about 1 to about 10 mg, often from about 2 to about 8 mg. For example, the lozenge may contain about 2 mg, about 3 mg, about 4 mg, about 5 mg, about 6 mg, about 7 mg, about 8 mg, or about 9 mg of a silver salt such as silver acetate. The lozenge optionally may contain additional components, such as additional alkaloids and/or additional active agents.

Nicotine may be provided by any suitable source, such as by isolating nicotine from tobacco or by preparing nicotine synthetically according to known techniques. Tobacco contains the (S)-nicotine enantiomer only; therefore, enantiomerically pure (S)-nicotine usually is used. Other forms of nicotine that may be used include stereo-selective, non-stereo-selective, racemic mixtures, and nicotine derivatives. The amount of nicotine in the lozenge usually ranges from about 0.5 mg to about 6 mg, often from about 1 to about 5 mg, and more often from about 2 to about 4 mg.

In a third embodiment, a smoking cessation aid comprises a lozenge containing (i) at least one of anatabine, anabasine, and nornicotine, and (ii) a therapeutically effective amount of a silver salt. The amount of silver salt typically ranges from about 1 to about 10 mg, often from about 2 to about 8 mg. For example, the lozenge may contain about 2 mg, about 3 mg, about 4 mg, about 5 mg, about 6 mg, about 7 mg, about 8 mg, or about 9 mg of a silver salt such as silver acetate.

Anatabine, anabasine, and nornicotine are minor tobacco alkaloids. These compounds are commercially available. Alternatively, the compounds may be isolated from tobacco or synthesized according to known techniques. For example, anatabine may be synthesized as described in N. M. Deo et al., "Regioselective Alkylation of N-(diphenylmethylidine)-3-(aminomethyl-pyridine: A Simple Route to Minor Tobacco Alkaloids and Related Compounds," 1137-1141 (1995). Nornicotine may be synthesized as described in S. Brandange et al., "N-Vinyl as N-H Protecting Group: A Convenient Synthesis of Myosmine," Acta Chem. Scand., B30, No. 1, p. 93 (1976). The amount of the minor tobacco alkaloid(s) in the lozenge usually ranges from about 0.25 mg to about 4 mg, often from about 0.5 to about 2 mg.

In the second and third embodiments, the active agents may be combined with one or more pharmaceutical diluents or carriers to form a lozenge. Nonlimiting examples of diluents and carriers include talc, magnesium stearate, dicalcium phosphate, magnesium aluminum silicate, calcium sulfate, starch, lactose, acacia, methylcellulose, and functionally similar materials. The lozenges may be coated or otherwise compounded to further provide prolonged or delayed action.

The following examples are provided for illustrative purposes and should not be regarded as limiting the scope of the invention.

### Example 1

Tobacco powder was combined with excipients in the product bowl of a fluid bed granulator. A binder solution was prepared and sprayed into the granulator, and the mixture was dried. The resulting mixture was dry-blended with flavorants and silver acetate to form lozenges (∼250 mg). Table I identifies the binder solution, tobacco mixture, and dry blend components.

**Table I**

| Component | Amount (wt)% |
|---|---|
| Binder Solution | |
| Gum Acacia (Instagum) | 4.00 |
| Buffers | 1.00 |
| Tobacco and Excipients | |
| Tobacco | 25.00 |
| D-Mannitol | 22.52 |
| Calcium Carbonate Light | 17.22 |
| Povidone K-90 | 3.50 |
| Cellulose | 2.78 |
| Titanium Dioxide | 1.00 |
| Sweetener | 2.50 |
| Dry Blend Components | |
| Flavorants | 7.00 |
| Magnesium Stearate | 0.50 |
| Stearic Acid (Triple Pressed) | 1.00 |
| Flo-Guard | 1.00 |
| D-Mannitol | 10.02 |
| Silver Acetate | 0.968 |
| TOTAL | 100.00 |

### Example 2

Lozenges containing nicotine and silver acetate may be prepared by combining nicotine with excipients as in Example 1 in the product bowl of a fluid bed granulator. A binder solution as in Example 1 may be prepared and sprayed into the granulator, and the mixture dried. The resulting mixture may be dry-blended with flavorants and silver acetate as in Example 1 to form lozenges containing 1.5 mg nicotine and 2.4 mg silver acetate.

### Example 3

Lozenges containing anatabine and silver acetate may be prepared by combining anatabine with excipients as in Example 1 in the product bowl of a fluid bed granulator. A binder solution as in Example 1 may be prepared and sprayed into the granulator, and the mixture dried. The resulting mixture may be dry-blended with flavorants and silver acetate as in Example 1 to form lozenges containing 1 mg anatabine and 2.4 mg silver acetate.

The lozenges may be taken orally as needed to reduce cravings for smoking. The lozenges should be taken only for short duration, e.g., up to several weeks, to avoid prolonged silver exposure.

### Example 4

A randomized, double-blind, placebo-controlled, crossover, pilot study was conducted to evaluate the efficacy of a smoking cessation aid containing tobacco extract and silver acetate. Forty-three (43) subjects were selected based on being healthy male or female adults who (1) were between 21-72 years old, (2) were regular smokers who smoked at least one pack of cigarettes per day for at least 5 years, (3) had a score of ≥ 6.0 on the Fagerström Nicotine Tolerance Scale, and (4) had a general desire to quit smoking within 6 months.

Each randomized subject was given a lozenge with two active ingredients, nicotine and silver acetate ("Product A"), and a placebo lozenge which contained no tobacco, nicotine or silver acetate ("Product B"). Subjects smoked their own brand of cigarettes following the dissolution of both lozenges.

The Product A lozenge, which contained 2.0 mg nicotine and 6 mg silver acetate, was made from powdered Virginia-type cured tobacco compressed into a small lozenge. The amount of tobacco was such that there was about 2.0 mg (+/- 5%) of nicotine in each lozenge. The lozenge dissolves without any residual fiber and therefore there is minimal or no need to expectorate. The Product B lozenge was a placebo containing no tobacco, nicotine, or silver acetate.

During the 3-hour session, subjects orally administered the first study product in the form of a dissolvable lozenge. Following full dissolution of the lozenge, subjects smoked a first cigarette. After cigarette smoking, subjects completed the Cigarette Taste Rating Questionnaire (CTRQ) and Relative Taste Rating Scale (RTRS).

Subjects orally administered the second study product in the form of a dissolvable lozenge. Following full dissolution of the lozenge, subjects smoked a second cigarette. After cigarette smoking, subjects completed another CTRQ and RTRS.

Subjects were randomized into one of two possible treatment sequences (see Table II below). Subjects received both products during the 3-hour session on Day 1. There was a 90-minute wash out period between the two study product administrations during which subjects could have snacks and water.

**Table II**

| Treatment Sequence | Product on Day 1 | |
|---|---|---|
| 1 | Product A | Product B |
| 2 | Product B | Product A |

The primary efficacy objective of this study was to determine the effectiveness of silver salt on discouraging smoking by imparting an unfavorable taste to the smoker, when a cigarette was smoked. Efficacy assessments consisted of the CTRQ and the RTRS. Table III summarizes the results of the taste components of the CTRQ.

**Table III**

| Question | Response | Product A | Product B | Treatment Group Comparison p-value^{a} |
|---|---|---|---|---|
| The overall taste of my cigarette is good. | Disagree | 79.1% | 16.3% | P<0.0001 |
| | Neither | 11.6% | 14.0% | |
| | Agree | 9.3% | 69.8% | |
| The aftertaste of my cigarette is good. | Disagree | 74.4% | 18.6% | p<0.0001 |
| | Neither | 18.6% | 18.6% | |
| | Agree | 7.0% | 62.8% | |
| The taste of my cigarette is normal. | Disagree | 88.3% | 21.0% | p<0.0001 |
| | Neither | 4.7% | 9.3% | |
| | Agree | 7.0% | 69.7% | |
| My cigarette has a strange taste. | Disagree | 32.6% | 65.1% | p=0.0037 |
| | Neither | 7.0% | 16.3% | |
| | Agree | 60.5% | 18.6% | |

| | | | | |
|---|---|---|---|---|
| ^{a}p-value based on Wilcoxon signed-rank test based on the paired differences | | | | |

The majority of subjects reported disliking the taste of their cigarette following administration of Product A on the CTRQ: 79.1 % disagreed that the overall taste of their cigarette was good and 74.4% disagreed that the aftertaste of their cigarette was good. In addition, most subjects reported a change in the taste of their cigarette: 88.3% disagreed that their cigarette tasted normal and 60.5% agreed that their cigarette had a strange taste.

Table IV summarizes the results of the satisfaction components of the CTRQ.

| Question | Response | Product A | Product B | Treatment Group Comparison |
|---|---|---|---|---|
| | | | | p-value^{a} |
| My cigarette is satisfactory. | Disagree | 81.4% | 11.6% | p<0.0001 |
| | Neither | 9.3% | 14.0% | |
| | Agree | 9.3% | 74.5% | |
| My cigarette is able to lessen my craving. | Disagree | 32.5% | 13.9% | p=0.0009 |
| | Neither | 23.3% | 14.0% | |
| | Agree | 44.2% | 72.1% | |
| Overall, I enjoyed my cigarette. | Disagree | 65.1% | 20.9% | p<0.0001 |
| | Neither | 23.3% | 7.0% | |
| | Agree | 11.6% | 72.1% | |

| | | | | |
|---|---|---|---|---|
| ^{a} p-value based on Wilcoxon signed-rank test based on the paired differences | | | | |

Overall, most subjects were not satisfied with and did not enjoy their cigarette following administration of the Product A lozenge: 81.4% disagreed that their cigarette was satisfactory, and 65.1% disagreed that they enjoyed their cigarette. In contrast, following administration of the Product B placebo lozenge, the majority of subjects agreed that their cigarette was satisfactory and that they enjoyed their cigarette, 74.5% and 72.1%, respectively.

Finally, fewer subjects (44.2% versus 72.1%) agreed that their cigarette was able to lessen their craving following administration of the Product A lozenge than with the Product B placebo lozenge. Statistical pair-wise analysis of the CTRQ data for Product A and Product B lozenges showed that the lozenges were highly, significantly different from each other, p<0.004 or less for all 7 questions of the CTRQ.

The results for the RTRS further support the results of the CTRQ. Table V summarizes the responses to the RTRS by treatment group.

**Table V**

| Taste of Cigarette Relative to Usual Cigarette | Product A | Product B | Treatment Group Comparison |
|---|---|---|---|
| | | | p-value^{a} |
| Worse | 90.7% | 37.2% | p<0.0001 |
| The Same | 4.7% | 34.9% | |
| Better | 4.6% | 27.9% | |

| | | | |
|---|---|---|---|
| ^{a}p-value based on Wilcoxon signed-rank test based on the paired differences | | | |

Consistent with the results of the taste components of the CTRQ, most subjects reported negative ratings for their cigarette following administration of the Product A lozenge. The majority of subjects reported that their cigarette tasted "Worse" (either "very much worse," "much worse," or "a little worse") following administration of the Product A lozenge (90.7%) compared to the Product B placebo (37.2%). Only 2 subjects (4.6%) reported that their cigarette tasted "Better" (either "very much better," "much better," or "a little better") following administration of the Product A lozenge.

As with the CTRQ, statistical pair-wise comparison of the RTRS results for the Product A and Product B lozenges showed that the two lozenges are highly, significantly different from each other, p<0.0001. The Product A lozenge imparted an unfavorable taste to the subjects. T abulation of the responses to the CTRQ showed that 4 times more subjects disliked the taste and aftertaste of their cigarette and that 3-4 times more subjects felt that their cigarette did not taste normal and had a strange taste following administration of the Product A lozenge compared to the Product B placebo lozenge. These results were confirmed by the RTRS, in which nearly 2.5 times more subjects reported a worsening of the taste of their cigarette following administration of the Product A lozenge than the Product B placebo lozenge.

The unfavorable taste of cigarettes following the Product A lozenge impacted the subjects' satisfaction with their cigarettes. Significantly more subjects were less satisfied with their cigarettes following the Product A lozenge compared to placebo - more than 7 times more subjects disagreed that their cigarette was satisfactory, and more than 3 times more subjects disagreed that they enjoyed their cigarette. These high dissatisfaction results are expected to discourage smokers from smoking.

### Example 5

A randomized, double-blind, active-controlled, crossover, pilot study was conducted to evaluate the efficacy of a smoking cessation aid containing tobacco extract and silver acetate. Thirty-seven (37) subjects were selected based on the same criteria described above in Example 4.

Each completed subject was given a lozenge with two active ingredients, nicotine and silver acetate ("Product A"), and a lozenge which contained powdered tobacco but no silver acetate ("Product C"). Subjects smoked their own brand of cigarettes.

The Product A lozenge, which contained 2.0 mg nicotine and 6 mg silver acetate, was made from powdered Virginia-type cured tobacco compressed into a small lozenge. The amount of tobacco was such that there was about 2.0 mg (+/- 5%) of nicotine in each lozenge. The Product C lozenge was Ariva^{®} Wintergreen (2.0 mg nicotine) (Star Scientific, Inc.), which contains powdered Virginia-type tobacco compressed into a small lozenge.

During the 5-hour session, the subjects orally administered the first study product in the form of a dissolvable lozenge. Following full dissolution of the lozenge, subjects smoked their first cigarette. After cigarette smoking, subjects completed the Cigarette Taste Rating Questionnaire (CTRQ) and Relative Taste Rating Scale (RTRS).

Subjects orally administered the second study product in the form of a dissolvable lozenge. Following full dissolution of the lozenge, subjects smoked their second cigarette. After cigarette smoking, subjects completed another CTRQ and RTRS. Subjects were discharged from the study at the end of the 5-hour session.

The primary efficacy objective of this study was to determine the effectiveness of silver salt in the Product A lozenge on discouraging smoking by imparting an unfavorable taste to the smoker, when a cigarette was smoked. Efficacy assessments consisted of the CTRQ and the RTRS. Table VI summarizes the results of the taste components of the CTRQ.

**Table VI**

| Question | Response | Product A | Product C | Treatment Group Comparison |
|---|---|---|---|---|
| | | | | p-value^{a} |
| The overall taste of my cigarette is good. | Disagree | 70.3% | 51.4% | p=0.0513 |
| | Neither | 8.1% | 13.5% | |
| | Agree | 21.6% | 35.1% | |
| The aftertaste of my cigarette is good. | Disagree | 73.0% | 45.9% | p=0.0036 |
| | Neither | 13.5% | 29.7% | |
| | Agree | 13.5% | 24.3% | |
| The taste of my cigarette is normal. | Disagree | 73.0% | 56.8% | p=0.0179 |
| | Neither | 5.4% | 5.4% | |
| | Agree | 21.6% | 37.8% | |
| My cigarette has a strange taste. | Disagree | 27.0% | 37.8% | p=0.1910 |
| | Neither | 16.2% | 16.2% | |
| | Agree | 56.8% | 45.9% | |

| | | | | |
|---|---|---|---|---|
| ^{a}p-value based on Wilcoxon signed-rank test based on the paired differences | | | | |

More subjects reported disliking the taste of their cigarette following administration of the Product A lozenge versus the Product C lozenge on the CTRQ: 70.3% versus 51.4% disagreed that the overall taste of their cigarette was good, and 73.0% versus 45.9% disagreed that the aftertaste of their cigarette was good, respectively. Similarly, more subjects reported a change in the taste of their cigarette following administration of the Product A lozenge versus the Product C lozenge: 73.0% versus 56.8% disagreed that their cigarette tasted normal and 56.8% versus 45.9% agreed that their cigarette had a strange taste, respectively.

Table VII summarizes the results of the satisfaction components of the CTRQ.

**Table VII**

| Question | Response | Product A | Product C | Treatment Group Comparison |
|---|---|---|---|---|
| | | | | p-value^{a} |
| My cigarette is very satisfactory. | Disagree | 64.9% | 45.9% | P=0.0237 |
| | Neither | 10.8% | 24.3% | |
| | Agree | 24.3% | 29.7% | |
| My cigarette is able to lessen my craving. | Disagree | 40.5% | 16.2% | P=0.0046 |
| | Neither | 29.7% | 37.8% | |
| | Agree | 29.7% | 45.9% | |
| 7. Overall, I enjoyed my cigarette. | Disagree | 59.5% | 45.9% | P=0.0805 |
| | Neither | 13.5% | 10.8% | |
| | Agree | 27.0% | 43.2% | |

| | | | | |
|---|---|---|---|---|
| ^{a} p-value based on Wilcoxon signed-rank test based on the paired differences | | | | |

Overall, most subjects were not satisfied with and did not enjoy their cigarette following administration of the Product A lozenge versus the Product C lozenge: 64.9% versus 45.9% disagreed that their cigarette was satisfactory, and 59.5% versus 45.9% disagreed that they enjoyed their cigarette, respectively.

Finally, fewer subjects (29.7% versus 45.9%) agreed that their cigarette was able to lessen their craving following administration of the Product A lozenge than with the Product C lozenge, respectively.

Pair-wise statistical analysis of the CTRQ results showed that the Product A and Product C lozenges were statistically significantly different from each other, p<0.025 or less for most of the taste and satisfaction questions of the CTRQ. The results for the RTRS support the results of the CTRQ. Table VIII summarizes the responses to the RTRS by treatment group.

**Table VIII**

| Taste of Cigarette Relative to Usual Cigarette | Product A | Product C | Treatment Group Comparison p-value^{a} |
|---|---|---|---|
| Worse | 81.1% | 67.6% | p=0.0201 |
| The Same | 18.9% | 29.7% | |
| Better | 0 | 2.7% | |

| | | | |
|---|---|---|---|
| ^{a}p-value based on Wilcoxon signed-rank test based on the paired differences | | | |

Consistent with the results of the taste components of the CTRQ, more subjects reported negative ratings for their cigarette following administration of the Product A lozenge. Significantly more subjects (p=0.0201) reported that their cigarette tasted "Worse" (either "very much worse," "much worse," or "a little worse") following administration of the Product A lozenge (81.1%) compared to the Product C lozenge (67.6%). No subjects reported that their cigarette tasted "Better" (either "very much better," "much better," or "a little better") following administration of the Product A lozenge.

The efficacy results of this study clearly showed that the Product A lozenge imparted an unfavorable taste to the subjects. Tabulation of the responses to the CTRQ showed that 37% and 59% more subjects disliked the taste and aftertaste of their cigarette, respectively, and that 29% and 24% more subjects felt that their cigarette did not taste normal and had a strange taste, respectively, following administration of the Product A lozenge compared to the Product C lozenge. These results were confirmed by the RTRS, in which 20% more subjects reported a worsening of the taste of their cigarette following administration of the Product A lozenge than the Product C lozenge.

The unfavorable taste of cigarettes following the Product A lozenge impacted the subjects' satisfaction with their cigarettes. More subjects were less satisfied with their cigarettes following the Product A lozenge compared to the Product C lozenge - 41% more subjects disagreed that their cigarette was satisfactory, and 29% more subjects disagreed that they enjoyed their cigarette. These high dissatisfaction results are expected to discourage smokers from smoking.

Finally, 35% fewer subjects indicated that their cigarette was able to lessen their craving, and 2.5 times more subjects disagreed that their cigarette was able to lessen their craving following Product A lozenge compared to the Product C lozenge.

### Example 6

This example reports a single center, randomized, double-blind, crossover study that assessed the effectiveness a tobacco-based lozenge product containing nicotine (2 mg) and silver acetate (6 mg) as active ingredients, on the craving for a cigarette compared to a lozenge containing only 6 mg silver acetate. Study subjects (n = 111 study completers) were 49% female, 93% Caucasian, and an average age of 44 years (range: 24-72). Subjects completed a series of craving and withdrawal questionnaires at three time points relative to oral administration of each study product: baseline, pre-administration, and post-administration. The primary measure of efficacy was the Questionnaire of Smoking Urges (QSU).

The Product A lozenge contained 2 mg nicotine (from powdered Virginia-type cured tobacco) and 6 mg silver acetate. The Product D lozenge contained 6 mg silver acetate, but no nicotine or tobacco. Each of the two products also contained flavorings.

FIG. 1 graphically depicts the subjects' decrease in craving for smoking following administration of the Product A lozenge containing tobacco and silver acetate and the Product D lozenge containing only silver acetate. Results from the QSU measure show that subjects reported a significant (p<0.0001) decrease in craving score following administration of Product A relative to Product D, as shown in Table IX below.

**Table IX: Mean (SD) Questionnaire of Smoking Urges (QSU) Total Scores and Mean (SD) Change in Scores from Pre- to Post-Treatment**

| | N | PreTreatment | Post-Treatment | Change | p-value (Pre vs. Post) | p-value (Prod. A vs. Prod. D) |
|---|---|---|---|---|---|---|
| Product A | 111 | 49.7 (14.8) | 38.0 (16.3) | -11.7 (13.8) | < 0.0001 | 0.003 |
| Product D | 111 | 49.0 (15.2) | 41.7 (16.8) | -7.3 (13.2) | < 0.0001 | |

| | | | | | | |
|---|---|---|---|---|---|---|
| SD = Standard deviation | | | | | | |

These results indicate that the Product A lozenge containing 2 mg nicotine (from powdered tobacco) and 6 mg silver acetate as active ingredients can significantly reduce craving for a cigarette in a sample population of highly nicotine-dependent, daily smokers. The Product A lozenge was found to significantly reduce craving relative to the Product D lozenge which contained silver acetate and flavoring, but no nicotine or tobacco.

## Claims

1. A smoking cessation aid comprising a lozenge containing (i) powdered tobacco, anatabine, nicotine, anabasine or nornicotine and (ii) a therapeutically effective amount of a silver salt.

2. The smoking cessation aid of claim 1 wherein the silver salt is silver acetate.

3. The smoking cessation aid of claim 2 wherein silver acetate is present in an amount from about 1 to about 10 mg.

4. The smoking cessation aid of claim 1 wherein the lozenge contains powdered tobacco.

5. The smoking cessation aid of claim 4 wherein the powdered tobacco consists essentially of Virginia flue cured tobacco, Burley tobacco or dark-fired tobacco.

6. The smoking cessation aid of claim 4 wherein the powdered tobacco comprises a blend of at least two varieties selected from the group consisting of Virginia flue cured tobacco, Burley tobacco, and dark-fired tobacco.

7. The smoking cessation aid of claim 4 wherein said powdered tobacco comprises pulverized tobacco.

8. The smoking cessation aid of claim 4 wherein said powdered tobacco is prepared from an extract of tobacco.

9. The smoking cessation aid of claim 1 wherein the lozenge contains at least one of anatabine, anabasine and nornicotine.

10. The smoking cessation aid of claim 9 wherein anatabine is present in an amount from about 0.25 to about 4 mg.

11. The smoking cessation aid of claim 9 wherein anabasine is present in an amount from about 0.25 to about 4 mg.

12. The smoking cessation aid of claim 9 wherein nornicotine is present in an amount from about 0.25 to about 4 mg.

13. The smoking cessation aid of claim 1 wherein the lozenge contains nicotine.

14. The smoking cessation aid of claim 13 wherein nicotine is present in an amount from about 1 to about 5 mg.

15. The smoking cessation aid of claim 13 wherein nicotine is present in an amount from about 2 to about 4 mg.

## Patentansprüche

1. Hilfsmittel zur Raucherentwöhnung, umfassend eine Pastille, enthaltend (i) Tabakpulver, Anatabin, Nicotin, Anabasin oder Nornicotin und (ii) eine therapeutisch wirksame Menge eines Silbersalzes.

2. Hilfsmittel zur Raucherentwöhnung nach Anspruch 1, wobei das Silbersalz Silberacetat ist.

3. Hilfsmittel zur Raucherentwöhnung nach Anspruch 2, wobei das Silberacetat in einer Menge von etwa 1 bis etwa 10 mg vorliegt.

4. Hilfsmittel zur Raucherentwöhnung nach Anspruch 1, wobei die Pastille Tabakpulver enthält.

5. Hilfsmittel zur Raucherentwöhnung nach Anspruch 4, wobei das Tabakpulver im Wesentlichen aus Flue Cured Virginia-Tabak, Burley-Tabak oder Dark-Fired Tabak besteht.

6. Hilfsmittel zur Raucherentwöhnung nach Anspruch 4, wobei das Tabakpulver ein Blend aus wenigstens zwei Sorten umfasst, ausgewählt aus der Gruppe bestehend aus Flue Cured Virginia-Tabak, Burley-Tabak und Dark-Fired Tabak besteht.

7. Hilfsmittel zur Raucherentwöhnung nach Anspruch 4, wobei das Tabakpulver gemahlenen Tabak umfasst.

8. Hilfsmittel zur Raucherentwöhnung nach Anspruch 4, wobei das Tabakpulver aus einem Tabakextrakt hergestellt ist.

9. Hilfsmittel zur Raucherentwöhnung nach Anspruch 1, wobei die Pastille wenigstens eines von Anatabin, Anabasin und Nornicotin enthält.

10. Hilfsmittel zur Raucherentwöhnung nach Anspruch 9, wobei Anatabin in einer Menge von etwa 0,25 bis etwa 4 mg vorliegt.

11. Hilfsmittel zur Raucherentwöhnung nach Anspruch 9, wobei Anabasin in einer Menge von etwa 0,25 bis etwa 4 mg vorliegt.

12. Hilfsmittel zur Raucherentwöhnung nach Anspruch 9, wobei Nornicotin in einer Menge von etwa 0,25 bis etwa 4 mg vorliegt.

13. Hilfsmittel zur Raucherentwöhnung nach Anspruch 1, wobei die Pastille Nicotin enthält.

14. Hilfsmittel zur Raucherentwöhnung nach Anspruch 13, wobei Nicotin in einer Menge von etwa 1 bis etwa 5 mg vorliegt.

15. Hilfsmittel zur Raucherentwöhnung nach Anspruch 13, wobei Nicotin in einer Menge von etwa 2 bis etwa 4 mg vorliegt.

## Revendications

1. Aide à la désaccoutumance du tabac, comprenant une pastille contenant (i) du tabac en poudre, de l'anatabine, de la nicotine, de l'anabasine ou de la nomicotine et (ii) une quantité thérapeutiquement efficace d'un sel d'argent.

2. Aide à la désaccoutumance du tabac selon la revendication 1, dans laquelle le sel d'argent est l'acétate d'argent.

3. Aide à la désaccoutumance du tabac selon la revendication 2, dans laquelle l'acétate d'argent est présent en une quantité d'environ 1 mg à environ 10 mg.

4. Aide à la désaccoutumance du tabac selon la revendication 1, dans laquelle la pastille contient du tabac en poudre.

5. Aide à la désaccoutumance du tabac selon la revendication 4, dans laquelle le tabac en poudre est essentiellement constitué d'un tabac de Virginie séché à l'air chaud, d'un tabac de la variété Burley ou d'un tabac séché au feu.

6. Aide à la désaccoutumance du tabac selon la revendication 4, dans laquelle le tabac en poudre comprend un mélange d'au moins deux variétés choisies dans le groupe constitué d'un tabac de Virginie séché à l'air chaud, d'un tabac de la variété Burley et d'un tabac séché au feu.

7. Aide à la désaccoutumance du tabac selon la revendication 4, dans laquelle ledit tabac en poudre comprend du tabac pulvérisé.

8. Aide à la désaccoutumance du tabac selon la revendication 4, dans laquelle ledit tabac en poudre est préparé à partir d'un extrait de tabac.

9. Aide à la désaccoutumance du tabac selon la revendication 1, dans laquelle la pastille contient au moins une parmi l'anatabine, l'anabasine et la nornicotine.

10. Aide à la désaccoutumance du tabac selon la revendication 9, dans laquelle l'anatabine est présente en une quantité d'environ 0,25 mg à environ 4 mg.

11. Aide à la désaccoutumance du tabac selon la revendication 9, dans laquelle l'anabasine est présente en une quantité d'environ 0,25 mg à environ 4 mg.

12. Aide à la désaccoutumance du tabac selon la revendication 9, dans laquelle la nornicotine est présente en une quantité d'environ 0,25 mg à environ 4 mg.

13. Aide à la désaccoutumance du tabac selon la revendication 1, dans laquelle la pastille contient de la nicotine.

14. Aide à la désaccoutumance du tabac selon la revendication 13, dans laquelle la nicotine est présente en une quantité d'environ 1 mg à environ 5 mg.

15. Aide à la désaccoutumance du tabac selon la revendication 13, dans laquelle la nicotine est présente en une quantité d'environ 2 mg à environ 4 mg.
